# EUROPEAN PATENT APPLICATION

(11) **EP 4 349 984 A1**
(43) Date of publication of application: **10.04.2024**
(21) Application number: 22837580.4
(22) Date of filing: 30.06.2022
(51) Int. Cl.: C12N 15/09, C12Q 1/6813, C12Q 1/6888

(54) **PROBE, PROBE SET, AND NUCLEIC ACID DETECTION KIT FOR MAMMALS**

(30) Priority: 08.07.2021 JP 2021113260
(71) Applicant: Kyushu University, National University Corporation, Nishi-ku Fukuoka-shi Fukuoka 819-0395 (JP)
(72) Inventor: OHKAWA, Yasuyuki, Fukuoka-shi, Fukuoka 819-0395 (JP); FUJII, Takeru, Fukuoka-shi, Fukuoka 819-0395 (JP)
(74) Representative: EP&C
(86) International application number: PCT/JP2022/026162
(87) International publication number: WO 2023/282164

(57) **Abstract**

A probe has a base sequence that does not hybridize with a nucleic acid of a mammal under stringent conditions, and has a sequence including a base sequence set forth in any of SEQ ID NOs:1 to 673. A probe set includes two or more kinds of the probes, and the two or more kinds of the probes do not hybridize with each other under stringent conditions.

## Description

### [Technical Field]

The present invention relates to a probe, a probe set, and a nucleic acid detection kit for mammals.

Priority is claimed on Japanese Patent Application No. 2021-113260, filed July 8, 2021, the content of which is incorporated herein by reference.

### [Background Art]

In recent years, transcriptome analysis is increasingly gaining importance in order to understand the relationship between expression changes in gene mutation and complex diseases such as cancer, diabetes, and heart disease. In Fluorescence In Situ Hybridization (FISH) methods, which are one of the techniques of transcriptome analysis, the expression distribution and the expression level of a specific nucleic acid in tissues and cells can be detected by using fluorescence.

Among the FISH methods, a sequential Fluorescence In Situ hybridization (seqFISH) method is a technology capable of directly identifying many RNA transcripts in a single cell while maintaining spatial localization positions. In the seqFISH method, first, in order to read a sequential barcode of each transcript, the transcript is labeled with a fluorescent probe by consecutive rounds of hybridization, and then the sequential barcodes are decoded to uniquely identify the RNA transcripts present in a single cell (see, for example, Patent Document 1). However, in the seqFISH method, the limit of optical resolution and the density of transcripts in a single cell have become problems. As a method for solving these problems, a seqFISH+ method, which can image mRNAs of 10,000 genes in a single cell with high accuracy at a resolution equal to or lower than the diffraction limit by using a standard confocal microscope, has been developed (see, for example, Non-Patent Document 1).

When detecting nucleic acids of mammals by the FISH methods described in Patent Document 1, Non-Patent Document 1, and the like, it is necessary to develop probes having sequences that are not present in the genomes of mammals. It is extremely difficult to design such a probe, and there are only several tens of types of probes that are currently published. Therefore, the number of genes that can be simultaneously detected is limited to about 20 to 30.

### [Citation List]

### [Patent Document]

[Patent Document 1]
Published Japanese Translation No. 2016-518843 of the PCT International Publication

### [Non-Patent Document]

[Non-Patent Document 1]
Eng C-H L., et al., "Transcriptome-scale super-resolved imaging in tissues by RNA seqFISH+.", Nature, Vol. 568, Issue 7751, pp. 235-239, 2019.

### [Summary of Invention]

### [Technical Problem]

The present invention was made in view of the above-described circumstances and provides a probe having a base sequence that is not present in the genomes of mammals.

### [Solution to Problem]

That is, the present invention includes the following aspects.
(1) A probe including:
   a base sequence that does not hybridize with a nucleic acid of a mammal under stringent conditions,
   wherein the probe has a sequence including a base sequence set forth in any of SEQ ID NOs:1 to 673.
(2) The probe according to (1),
   in which the mammal is one or more kinds of animals selected from the group consisting of a human and a mouse.
(3) The probe according to (1) or (2),
   in which the mammal is one or more kinds of animals selected from the group consisting of a human, a mouse, a zebrafish, an African clawed frog, and a rat, and
   the probe has a sequence including a base sequence set forth in any of SEQ ID NOs:6, 22, 31, 42, 43, 54, 61, 71, 86 to 88, 91, 109, 114, 118, 125, 128, 134, 140, 141, 146, 149, 151, 157, 167, 201, 213, 214, 218, 220, 221, 228, 230, 234, 241, 242, 248, 253, 283, 289, 294, 298, 303, 305, 310, 327, 349, 363, 365, 375, 378, 385, 394, 400, 406, 412, 416, 418, 420, 421, 430, 431, 439, 475, 501, 523, 532, 548, 557, 563, 585, 589, 593, 608, 620, 631, 642, and 646.
(4) The probe according to any one of (1) to (3), further including:
   a marker substance.
(5) A probe set including:
   two or more kinds of the probes according to any one of (1) to (4),
   in which the two or more kinds of the probes do not hybridize with each other under stringent conditions.
(6) A nucleic acid detection kit for mammals, the nucleic acid detection kit including:
   one or more kinds of detection probes including the probe according to (4); and
   one or more kinds of capturing probes each having a sequence A complementary to at least a portion of the detection probes and a sequence B complementary to at least a portion of a target nucleic acid of a mammal,
   in which in a case where the nucleic acid detection kit includes two or more kinds of the detection probes, the two or more kinds of the detection probes do not hybridize with each other under stringent conditions, and
   in a case where the nucleic acid detection kit includes two or more kinds of the capturing probes, the two or more kinds of the capturing probes do not hybridize with each other under stringent conditions.
(7) A method for simultaneously detecting a plurality of nucleic acids of a mammal, using the nucleic acid detection kit for mammals according to (6), the method including:
   bringing two or more kinds of the capturing probes into contact with a cell or a tissue of a mammal;
   removing the unhybridized capturing probes by washing and then bringing two or more kinds of the detection probes into contact with a cell or a tissue of a mammal; and
   removing the unhybridized detection probes by washing and then detecting the detection probes.

### [Advantageous Effects of Invention]

According to the probe of the above-described aspect, a probe having a base sequence that is not present in a genome of a mammal can be provided.

### [Brief Description of Drawings]

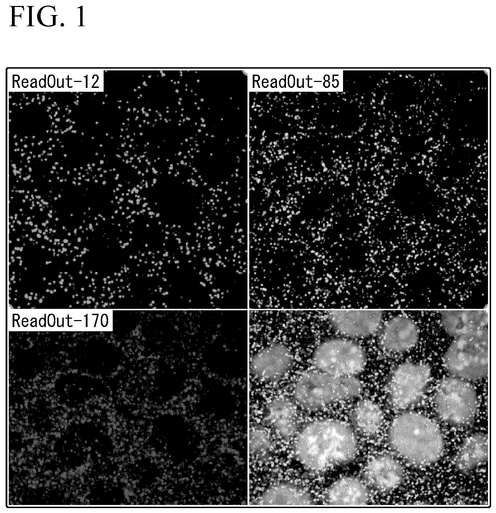

FIG. 1 is fluorescence images in which mRNA in mouse ES cells is visualized by an smFISH method using three kinds of probes in Example 2.

### [Description of Embodiments]

### <Probe>

A probe of the present embodiment has a base sequence that does not hybridize with a nucleic acid of a mammal under stringent conditions and has a sequence including a base sequence set forth in any of SEQ ID NOs:1 to 673.

As shown in Examples that will be described later, the inventors carried out screening of probes having base sequences that are not present in the whole genome sequences of mammals (particularly, a human and a mouse) from a probe library designed by a unique algorithm, thereby completing the present invention. The base sequences set forth in SEQ ID NOs:1 to 673 each consist of 15 bases and are all base sequences that are not present in the whole genome sequences of a human and a mouse, and the inventors have found the base sequences for the first time here.

In addition, as shown in the Examples that will be described later, among the above-described 673 types of probes, base sequences set forth in SEQ ID NOs:6, 22, 31, 42, 43, 54, 61, 71, 86 to 88, 91, 109, 114, 118, 125, 128, 134, 140, 141, 146, 149, 151, 157, 167, 201, 213, 214, 218, 220, 221, 228, 230, 234, 241, 242, 248, 253, 283, 289, 294, 298, 303, 305, 310, 327, 349, 363, 365, 375, 378, 385, 394, 400, 406, 412, 416, 418, 420, 421, 430, 431, 439, 475, 501, 523, 532, 548, 557, 563, 585, 589, 593, 608, 620, 631, 642, and 646 each consist of 15 bases and are all base sequences that are not present in the whole genome sequences of a human, a mouse, a zebrafish, an African clawed frog, and a rat, and the inventors have found the base sequences for the first time here.

The term "probe" means a molecule or molecular complex that is used to detect a nucleic acid. Since the probe of the present embodiment has a base sequence that does not hybridize with a nucleic acid of a mammal under stringent conditions, the probe is suitably used as a detection probe for binding to a capturing probe when detecting a nucleic acid of a mammal and detecting the nucleic acid of a mammal, as will be described later. That is, a probe having a sequence including a base sequence set forth in any of SEQ ID NOs:1 to 673 is suitably used as a detection probe for detecting a nucleic acid of a human or a mouse. In addition, a probe having a sequence including a base sequence set forth in any of SEQ ID NOs:6, 22, 31, 42, 43, 54, 61, 71, 86 to 88, 91, 109, 114, 118, 125, 128, 134, 140, 141, 146, 149, 151, 157, 167, 201, 213, 214, 218, 220, 221, 228, 230, 234, 241, 242, 248, 253, 283, 289, 294, 298, 303, 305, 310, 327, 349, 363, 365, 375, 378, 385, 394, 400, 406, 412, 416, 418, 420, 421, 430, 431, 439, 475, 501, 523, 532, 548, 557, 563, 585, 589, 593, 608, 620, 631, 642, and 646 is suitably used as a detection probe for detecting a nucleic acid of one or more kinds of mammals selected from the group consisting of a human and a mouse.

The phrase "under stringent conditions" refers to conditions under which a specific hybrid is formed while a non-specific hybrid is not formed. As a case under stringent conditions, more specifically, for example, conditions of allowing hybridization by performing incubation at 55°C or higher and 70°C or lower for several hours to overnight in a hybridization buffer formed from 5 × SSC (composition of 20 × SSC: 3 M sodium chloride and 0.3 M citric acid solution, pH 7.0), 0.1% by weight of N-lauroyl sarcosine, 0.02% by weight of SDS, 2% by weight of a blocking reagent for nucleic acid hybridization, and 50% formamide, may be mentioned. Incidentally, the washing buffer to be used at the time of washing after the incubation is preferably a 0.1% by weight SDS-containing 1 × SSC solution, and more preferably a 0.1% by weight SDS-containing 0.1 × SSC solution.

The nucleic acid constituting the probe of the present embodiment can have a length of, for example, 10 bases or more and 50 bases or fewer, and a nucleic acid having a length of 10 bases or more and 30 bases or fewer is preferred.

The nucleic acid constituting the probe of the present embodiment may further include another sequence in addition to a base sequence set forth in any of SEQ ID NOs:1 to 673; however, a nucleic acid having only a base sequence set forth in any of SEQ ID NOs:1 to 673 is preferred.

The term "nucleic acid" means a high-molecular weight organic compound in which nitrogen-containing bases derived from purine or pyrimidine, sugar, and phosphoric acid are regularly bonded, and nucleic acid analogs and the like are also included therein.

The nucleic acid constituting the probe of the present embodiment may be DNA or may be RNA. In addition, the probe of the present embodiment is not limited to a probe consisting of a natural nucleic acid, and the probe may include artificial nucleic acids such as BNA, ethylene-crosslinked nucleic acid (ENA), peptide nucleic acid (PNA), glycol nucleic acid (GNA), and threose nucleic acid (TNA).

The probe of the present embodiment may further have a marker substance. That is, the probe of the present embodiment is composed of a nucleic acid having a base sequence that does not hybridize with a nucleic acid of a mammal under stringent conditions and having a sequence including a base sequence set forth in any of SEQ ID NOs:1 to 673, and a marker substance.

The term "marker substance" means a substance that directly or indirectly produces a signal detectable by chemical means or physical means. Examples of the marker substance include enzyme markers such as peroxidases (for example, horseradish peroxidase) and alkaline phosphatase; fluorescent markers such as carboxyfluorescein (FAM), 6-carboxy-4',5'-dichloro 2',7'-dimethoxyfluorescein (JOE), fluorescein isothiocyanate (FITC), tetrachlorofluorescein (TET), 5'-hexachloro-fluorescein-CE phosphoramidite (HEX), Cy3, Cy5, Alexa 488, Alexa 555, Alexa 568, and Alexa 647; radioisotope markers such as Iodine 125; electrochemiluminescent markers such as a ruthenium complex; and metal nanoparticles; however, the examples are not limited to these. Preferred examples of the marker substance include fluorescent markers (fluorescent dyes).

In the probe of the present embodiment, the marker substance is bonded to the 5'-end or the 3'-end of the nucleic acid in the probe directly, or indirectly through a linker or the like.

The term "linker" means a linking moiety that links two substances, or a molecule that is used to link two substances.

### <Probe set>

A probe set of the present embodiment includes two or more kinds of the above-described probes, and the two or more kinds of the probes do not hybridize with each other under stringent conditions.

In the probe set of the present embodiment, since two or more kinds of the probes do not hybridize with each other under stringent conditions, a plurality of target nucleic acids can be simultaneously detected with high accuracy.

The probe set of the present embodiment includes two or more kinds of the above-mentioned probes, preferably 10 or more kinds thereof, more preferably 50 or more kinds thereof, even more preferably 100 or more kinds thereof, particularly preferably 200 or more kinds thereof, and most preferably 240 or more kinds thereof. When the probe set of the present embodiment includes the above-mentioned probes of a number of types equal to or more than the above-described lower limit value, a larger number of nucleic acids of a mammal can be simultaneously detected. Particularly, when the probe set includes 240 or more kinds of the above-mentioned probes, it is possible to simultaneously detect all mRNAs of a specific mammal (particularly, a human or a mouse) by smFISH.

In the probe set of the present embodiment, when the probes have marker substances, it is preferable that the two or more kinds of the probes have marker substances that are different from each other.

### <Nucleic acid detection kit for mammals>

The nucleic acid detection kit for a mammal of the present embodiment (hereinafter, may be simply referred to as "nucleic acid detection kit of the present embodiment") includes one or more kinds of detection probes and one or more kinds of capturing probes.

The detection probe consists of a probe having the above-mentioned marker substance. That is, the detection probe is composed of a nucleic acid having a base sequence that does not hybridize with a nucleic acid of a mammal under stringent conditions and having a sequence including a base sequence set forth in any of SEQ ID NOs:1 to 673, and a marker substance.

Examples of the marker substance include those mentioned as examples in the above-described section "Probe", and among these, a fluorescent marker (fluorescent dye) is preferred.

In addition, with regard to the nucleic acid detection kit of the present embodiment, when two or more kinds of detection probes are included, it is preferable that the two or more kinds of detection probes have marker substances that are different from each other.

The capturing probe has a sequence A complementary to at least a portion of the detection probe and a sequence B complementary to at least a portion of a target nucleic acid of a mammal.

When two or more kinds of the detection probes are included, the two or more kinds of the detection probes do not hybridize with each other under stringent conditions, and when two or more kinds of the capturing probes are included, the two or more kinds of capturing probes do not hybridize with each other under stringent conditions. As a result, a plurality of target nucleic acids can be detected simultaneously with high accuracy.

With regard to the nucleic acid detection kit of the present embodiment, the target nucleic acid is not particularly limited as long as it is derived from a mammal, and the target nucleic acid may be DNA or may be RNA.

Examples of the mammal include, but are not limited to, a human, a chimpanzee, and other primates; and livestock animals, pet animals, and experimental animals, such as a dog, a cat, a rabbit, a horse, a sheep, a goat, a cow, a pig, a rat (including a nude rat), a mouse (including a nude mouse and a scid mouse), a hamster, and a guinea pig, and the examples are not limited to these. Among them, a human or a mouse is preferred as the mammal from which the target nucleic acid is derived.

### [Capturing probe]

In the capturing probe, the sequence A consists of a sequence complementary to at least a portion of the detection probe. By having the sequence A, the capturing probe can hybridize with the detection probe under stringent conditions to detect the target nucleic acid.

From the viewpoint of binding stability with respect to the detection probe, it is preferable that the sequence A consists of a sequence complementary to a sequence having a length of about 10 or more consecutive bases and 50 or fewer consecutive bases in the nucleic acid constituting the detection probe, and it is particularly preferable that the sequence A consists of a sequence complementary to the base sequence set forth in any of SEQ ID NOs:1 to 673 in the nucleic acid constituting the detection probe.

The length of the sequence A can be set to, for example, 10 bases or more and 50 bases or fewer, and the length is preferably 10 bases or more and 30 bases or fewer.

In the capturing probe, the sequence B is a sequence complementary to at least a portion of the target nucleic acid of a mammal. By having the sequence B, the capturing probe can hybridize with the target nucleic acid under stringent conditions to capture the target nucleic acid.

The length of the sequence B can be set to, for example, 10 bases or more and 2000 bases or fewer or can be set to 50 bases or more and 1500 bases or fewer.

The nucleic acid constituting the capturing probe may be DNA or may be RNA. In addition, the capturing probe is not limited to a probe consisting of a natural nucleic acid, and the capturing probe may include artificial nucleic acids such as BNA, ethylene-crosslinked nucleic acid (ENA), peptide nucleic acid (PNA), glycol nucleic acid (GNA), and threose nucleic acid (TNA).

### [Other elements]

The nucleic acid detection kit of the present embodiment may include other elements in addition to the above-described detection probe and the above-described capturing probe. Examples of the other elements include a detection reagent for a marker substance, a reagent for sample preparation, a diluent, a buffer (washing buffer or the like), and an instruction for use.

The nucleic acid detection kit of the present embodiment can be utilized for a method for simultaneously detecting a plurality of nucleic acids of a mammal. Specifically, the nucleic acid detection kit can be used for transcriptome analysis by various ISH methods such as seqFISH, and for the detection of structural changes in the genome.

The method for simultaneously detecting a plurality of nucleic acids of a mammal is a method of using the above-described nucleic acid detection kit and includes:
bringing two or more kinds of the capturing probes into contact with a cell or a tissue of a mammal;
removing the unhybridized capturing probes by washing and then bringing two or more kinds of the detection probes into contact with a cell or a tissue of a mammal; and
removing the unhybridized detection probes by washing and then detecting the detection probes.

The capturing probe and the detection probe are diluted with various known buffer solutions and are added to cells or tissues of a mammal. The cell or tissue of a mammal may be a cell or tissue derived from the above-mentioned mammal, and in the case of mammals including a human, the method can be carried out in vitro, whereas in the case of mammals not including a human, the method can be carried out in vivo. That is, the cells or tissues as an object may be in a state of being immobilized, may be in a cultured state, or may be in a state of functioning in the living body.

The contact between the capturing probe and a cell or a tissue of a mammal can be carried out by incubating the system at 30°C or higher and 45°C or lower (preferably about 37°C) for 24 hours or longer and 60 hours or shorter (preferably 36 hours or longer and 48 hours or shorter).

The contact between the detection probe and a cell or a tissue of a mammal can be carried out by incubating at 30°C or higher and 45°C or lower (preferably about 42°C) for 10 hours or longer and 24 hours or shorter (preferably about 16 hours).

Washing of the capturing probe and the detection probe is performed once or more by using a known washing buffer.

A method for detecting the detection probe can be appropriately selected according to the type of the marker substance contained in the detection probe. Specifically, for example, when the marker substance is a fluorescent substance, a method of performing detection using a confocal microscope may be mentioned.

Therefore, according to the method for simultaneously detecting a plurality of nucleic acids of a mammal according to the present embodiment, a large number of various nucleic acids of a mammal can be detected simultaneously.

### [Examples]

Hereinafter, the present invention will be described by way of Examples; however, the present invention is not intended to be limited to the following Examples.

### [Example 1]

### (Production of probe)

The inventors have produced a probe library consisting of random base sequences designed by a unique algorithm. Next, for the base sequence of each probe in the produced probe library, the sequence identity with the whole genome sequences of a human and a mouse was analyzed with the online edition BLAST (a human, a mouse) using Genomic + transcript databases, and with Bowtie2 (a human, a mouse, a zebrafish, an African clawed frog, and a rat) using Refseq transcript data for the index, probes having base sequences that are not present in the whole genome sequences of a human, a mouse, a zebrafish, an African clawed frog, and a rat were screened.

As a result, 673 types of probes having the base sequences set forth in SEQ ID NOs: 1 to 673 were obtained as the probes having base sequences that are not present in the whole genome sequences of a human and a mouse.

Among these probes, 78 types of probes having base sequences set forth in SEQ ID NOs:6, 22, 31, 42, 43, 54, 61, 71, 86 to 88, 91, 109, 114, 118, 125, 128, 134, 140, 141, 146, 149, 151, 157, 167, 201, 213, 214, 218, 220, 221, 228, 230, 234, 241, 242, 248, 253, 283, 289, 294, 298, 303, 305, 310, 327, 349, 363, 365, 375, 378, 385, 394, 400, 406, 412, 416, 418, 420, 421, 430, 431, 439, 475, 501, 523, 532, 548, 557, 563, 585, 589, 593, 608, 620, 631, 642, and 646 were probes that are not present in the whole genome sequences of a zebrafish, an African clawed frog, and a rat as well, in addition to a human and a mouse.

### [Example 2]

### (Test for visualizing mRNA in mouse ES cells by smFlSH method using three kinds of probes)

Next, a test for visualizing mRNA in mouse ES cells was performed using the three types of probes shown in the following Table 1 among the probes obtained in the above-described Example 1, as detection probes. As fluorescent substances, products obtained by binding Alexa647 to ReadOut-12, Cy3B to ReadOut-85, and Alexa488 to ReadOut-170 were prepared.

**[Table 1]**

| Probe name | Base sequence (5' → 3') | SEQ ID NO: |
|---|---|---|
| ReadOut-12 | TCGCGAGTAGAGCAC | 12 |
| ReadOut-85 | AAGGCGATATACGAT | 85 |
| ReadOut-170 | TTCGCTAGTTCGTCT | 170 |

Regarding the procedure of the smFISH method, the method was carried out by a known technique. Specifically, first, mouse ES cells were cultured in advance on a slide glass, and the cells were immobilized by using 4 v/v% paraformaldehyde at room temperature for 10 minutes. Next, 70% ethanol was added to the sample, and the sample was incubated for 1 hour. Thereafter, the probes shown in the following Table 2-1 to Table 4-2, which target Dnmt3b mRNA, Adgrg6 mRNA, and Vgf mRNA, were added to the sample as capturing probes, and the sample was incubated at 37°C for 36 hours or longer and 48 hours or shorter to be hybridized.

**[Table 2-1]**

| Probe name | Base sequence (5' → 3') | SEQ ID NO: |
|---|---|---|
| Dnmt3b_1 | CGGCAAACTACGCGAGCCATACACGATACTGCGCATAGTGCTCTACTCGCGAACTCCTGATCTCCATC CTGGCTCAAGTCAACAGACGCGTGACGTAATATCCGGAGCTAATTCCTATAGTGAGTCGTATTAGCTT | 674 |
| Dnmt3b_2 | CGGCAAACTACGCGAGCCATACACGATACTGCGCATAGTGCTCTACTCGCGAACGCTTGGAGGTGGCT TTCCAGGACACCAACAGACGCGTGACGTAATATCCGGAGCTAATTCCTATAGTGAGTCGTATTAGCTT | 675 |
| Dnmt3b_3 | CGGCAAACTACGCGAGCCATACACGATACTGCGCATAGTGCTCTACTCGCGAACCCTCACAGCACACG GTGCAGTAGGACTACAGACGCGTGACGTAATATCCGGAGCTAATTCCTATAGTGAGTCGTATTAGCTT | 676 |
| Dnmt3b_4 | CGGCAAACTACGCGAGCCATACACGATACTGCGCATAGTGCTCTACTCGCGAAACCTCCAGACACTCC ACACAGAAGCATCACAGACGCGTGACGTAATATCCGGAGCTAATTCCTATAGTGAGTCGTATTAGCTT | 677 |
| Dnmt3b_5 | CGGCAAACTACGCGAGCCATACACGATACTGCGCATAGTGCTCTACTCGCGAAAGGGTTCCTGCAGCT TGGCATCCTCAGCACAGACGCGTGACGTAATATCCGGAGCTAATTCCTATAGTGAGTCGTATTAGCTT | 678 |
| Dnmt3b_6 | CGGCAAACTACGCGAGCCATACACGATACTGCGCATAGTGCTCTACTCGCGAAGGCAGCGCTGAGGGA GGCACATATAGCAACAGACGCGTGACGTAATATCCGGAGCTAATTCCTATAGTGAGTCGTATTAGCTT | 679 |
| Dnmt3b_7 | CGGCAAACTACGCGAGCCATACACGATACTGCGCATAGTGCTCTACTCGCGAAATACCCAACTCCTTG AGCACCAAGTACCACAGACGCGTGACGTAATATCCGGAGCTAATTCCTATAGTGAGTCGTATTAGCTT | 680 |
| Dnmt3b_8 | CGGCAAACTACGCGAGCCATACACGATACTGCGCATAGTGCTCTACTCGCGAAGATCTGGCCTTCATG CTTAACAGTTCCCACAGACGCGTGACGTAATATCCGGAGCTAATTCCTATAGTGAGTCGTATTAGCTT | 681 |
| Dnmt3b_9 | CGGCAAACTACGCGAGCCATACACGATACTGCGCATAGTGCTCTACTCGCGAACGTTAGAGAGATCAT TGCATGGGCTTCCACAGACGCGTGACGTAATATCCGGAGCTAATTCCTATAGTGAGTCGTATTAGCTT | 682 |
| Dnmt3b_10 | CGGCAAACTACGCGAGCCATACACGATACTGCGCATAGTGCTCTACTCGCGAAAAGTACCGGGCCCTG TGAGCAGCAGACAACAGACGCGTGACGTAATATCCGGAGCTAATTCCTATAGTGAGTCGTATTAGCTT | 683 |

**[Table 2-2]**

| Probe name | Base sequence (5' → 3') | SEQ ID NO: |
|---|---|---|
| Dnmt3b_11 | CGGCAAACTACGCGAGCCATACACGATACTGCGCATAGTGCTCTACTCGCGAAGAAGCCATCACGGGC CTGTTCATTCCGGACAGACGCGTGACGTAATATCCGGAGCTAATTCCTATAGTGAGTCGTATTAGCTT | 684 |
| Dnmt3b_12 | CGGCAAACTACGCGAGCCATACACGATACTGCGCATAGTGCTCTACTCGCGAATTTGCCCTGTCTGAT GGAGTTCGACTTGACAGACGCGTGACGTAATATCCGGAGCTAATTCCTATAGTGAGTCGTATTAGCTT | 685 |
| Dnmt3b_13 | CGGCAAACTACGCGAGCCATACACGATACTGCGCATAGTGCTCTACTCGCGAAACGTCGTCCTTGCCA TTCATGACTACAGACAGACGCGTGACGTAATATCCGGAGCTAATTCCTATAGTGAGTCGTATTAGCTT | 686 |
| Dmnt3b_14 | CGGCAAACTACGCGAGCCATACACGATACTGCGCATAGTGCTCTACTCGCGAAACAGGTGTCTGATGA CCGGTACACTCCAACAGACGCGTGACGTAATATCCGGAGCTAATTCCTATAGTGAGTCGTATTAGCTT | 687 |
| Dnmt3b_15 | CGGCAAACTACGCGAGCCATACACGATACTGCGCATAGTGCTCTACTCGCGAAAAGCAGCCAGGTCTG AGCTGTGCACACGACAGACGCGTGACGTAATATCCGGAGCTAATTCCTATAGTGAGTCGTATTAGCTT | 688 |
| Dnmt3b_16 | CGGCAAACTACGCGAGCCATACACGATACTGCGCATAGTGCTCTACTCGCGAAGGAGATCCCGCGTCT GGAAAGGTATATAACAGACGCGTGACGTAATATCCGGAGCTAATTCCTATAGTGAGTCGTATTAGCTT | 689 |
| Dnmt3b_17 | CGGCAAACTACGCGAGCCATACACGATACTGCGCATAGTGCTCTACTCGCGAAGCAGCATGAACCTGA GTACTAAGAGAGCACAGACGCGTGACGTAATATCCGGAGCTAATTCCTATAGTGAGTCGTATTAGCTT | 690 |
| Dnmt3b_18 | CGGCAAACTACGCGAGCCATACACGATACTGCGCATAGTGCTCTACTCGCGAATCCAGCAAATGTGGA GCACGGGTTACTTACAGACGCGTGACGTAATATCCGGAGCTAATTCCTATAGTGAGTCGTATTAGCTT | 691 |
| Dnmt3b_19 | CGGCAAACTACGCGAGCCATACACGATACTGCGCATAGTGCTCTACTCGCGAACCTTGCTCATCTGAG CCCACATTCACAAACAGACGCGTGACGTAATATCCGGAGCTAATTCCTATAGTGAGTCGTATTAGCTT | 692 |
| Dnmt3b_20 | CGGCAAACTACGCGAGCCATACACGATACTGCGCATAGTGCTCTACTCGCGAATGCAGCTTTCTTATC TCCAGTGATGCCCACAGACGCGTGACGTAATATCCGGAGCTAATTCCTATAGTGAGTCGTATTAGCTT | 693 |
| Dnmt3b_21 | CGGCAAACTACGCGAGCCATACACGATACTGCGCATAGTGCTCTACTCGCGAATTGACCTGCGGCCTC TGGTCTCTGGTGTACAGACGCGTGACGTAATATCCGGAGCTAATTCCTATAGTGAGTCGTATTAGCTT | 694 |
| Dnmt3b_22 | CGGCAAACTACGCGAGCCATACACGATACTGCGCATAGTGCTCTACTCGCGAATCCATCTCCTGTCAT GTCCTGCGTGTAAACAGACGCGTGACGTAATATCCGGAGCTAATTCCTATAGTGAGTCGTATTAGCTT | 695 |
| Dnmt3b_23 | CGGCAAACTACGCGAGCCATACACGATACTGCGCATAGTGCTCTACTCGCGAACTTGGTCTCACGGGT GAGCTTTGGCATTACAGACGCGTGACGTAATATCCGGAGCTAATTCCTATAGTGAGTCGTATTAGCTT | 696 |
| Dnmt3b_24 | CGGCAAACTACGCGAGCCATACACGATACTGCGCATAGTGCTCTACTCGCGAAGTAGCCGGAAACTCC ACAGGGTACTCCTACAGACGCGTGACGTAATATCCGGAGCTAATTCCTATAGTGAGTCGTATTAGCTT | 697 |
| Dnmt3b_25 | CGGCAAACTACGCGAGCCATACACGATACTGCGCATAGTGCTCTACTCGCGAATGTCACTTCTTCCAT GAAGTCGACGCTGACAGACGCGTGACGTAATATCCGGAGCTAATTCCTATAGTGAGTCGTATTAGCTT | 698 |

**[Table 3-1]**

| Probe name | Base sequence (5' → 3') | SEQ ID NO: |
|---|---|---|
| Adgrg6_1 | CGGCAAACTACGCGAGCCATAATCGTATATCGCCTTACGATAATAGAACCGAATGTGCCAGTCTATGACA TTCCCAGACACAGCGTATATAGTGGTTACGCCGTTACATCAGTCCTATAGTGAGTCGTATTAGCTT | 699 |
| Adgrg6_2 | CGGCAAACTACGCGAGCCATAATCGTATATCGCCTTACGATAATAGAACCGAACCACAGCTCAGGTTGCC CTCGGCTTTCAAGCGTATATAGTGGTTACGCCGTTACATCAGTCCTATAGTGAGTCGTATTAGCTT | 700 |
| Adgrg6_3 | CGGCAAACTACGCGAGCCATAATCGTATATCGCCTTACGATAATAGAACCGAACTTGAACAGTTGGAAGT GTCAACAGGGCAGCGTATATAGTGGTTACGCCGTTACATCAGTCCTATAGTGAGTCGTATTAGCTT | 701 |
| Adgrg6_4 | CGGCAAACTACGCGAGCCATAATCGTATATCGCCTTACGATAATAGAACCGAATTAGAGTGGAGCCGAGG GTGATGTCAATAGCGTATATAGTGGTTACGCCGTTACATCAGTCCTATAGTGAGTCGTATTAGCTT | 702 |
| Adgrg6_5 | CGGCAAACTACGCGAGCCATAATCGTATATCGCCTTACGATAATAGAACCGAAGATGAGACTCCAAGGGC CAAATTCTGTGAGCGTATATAGTGGTTACGCCGTTACATCAGTCCTATAGTGAGTCGTATTAGCTT | 703 |
| Adgrg6_6 | CGGCAAACTACGCGAGCCATAATCGTATATCGCCTTACGATAATAGAACCGAAGGATCTGTCTGTCCGTT CCCGAAGTCCAAGCGTATATAGTGGTTACGCCGTTACATCAGTCCTATAGTGAGTCGTATTAGCTT | 704 |
| Adgrg6_7 | CGGCAAACTACGCGAGCCATAATCGTATATCGCCTTACGATAATAGAACCGAATCAAGCTCCACCGAAGA AGATCCCGGCTAGCGTATATAGTGGTTACGCCGTTACATCAGTCCTATAGTGAGTCGTATTAGCTT | 705 |
| Adgrg6_8 | CGGCAAACTACGCGAGCCATAATCGTATATCGCCTTACGATAATAGAACCGAAAAGGTACCGGAAGGATT GGACAGGACAAAGCGTATATAGTGGTTACGCCGTTACATCAGTCCTATAGTGAGTCGTATTAGCTT | 706 |
| Adgrg6_9 | CGGCAAACTACGCGAGCCATAATCGTATATCGCCTTACGATAATAGAACCGAAGGCACTCCTTGGAAGAT CCATCAGAACTAGCGTATATAGTGGTTACGCCGTTACATCAGTCCTATAGTGAGTCGTATTAGCTT | 707 |
| Adgrg6_10 | CGGCAAACTACGCGAGCCATAATCGTATATCGCCTTACGATAATAGAACCGAATCCCACAGATCTGCACC ATGACCACAATAGCGTATATAGTGGTTACGCCGTTACATCAGTCCTATAGTGAGTCGTATTAGCTT | 708 |

**[Table 3-2]**

| Probe name | Base sequence (5' → 3') | SEQ ID NO: |
|---|---|---|
| Adgrg6_11 | CGGCAAACTACGCGAGCCATAATCGTATATCGCCTTACGATAATAGAACCGAACGCCTCCACTGTTTCT GAACATTCTCCTAGCGTATATAGTGGTTACGCCGTTACATCAGTCCTATAGTGAGTCGTATTAGCTT | 709 |
| Adgrg6_12 | CGGCAAACTACGCGAGCCATAATCGTATATCGCCTTACGATAATAGAACCGAACCCAGGTTATCGGAGC TCTTCTTGATGAAGCGTATATAGTGGTTACGCCGTTACATCAGTCCTATAGTGAGTCGTATTAGCTT | 710 |
| Adgrg6_13 | CGGCAAACTACGCGAGCCATAATCGTATATCGCCTTACGATAATAGAACCGAAGGACGGGAATGATTGA CGTCTGTTCTCCAGCGTATATAGTGGTTACGCCGTTACATCAGTCCTATAGTGAGTCGTATTAGCTT | 711 |
| Adgrg6_14 | CGGCAAACTACGCGAGCCATAATCGTATATCGCCTTACGATAATAGAACCGAAGACATTCCACCGGCGT AGCTTTCCGTGCAGCGTATATAGTGGTTACGCCGTTACATCAGTCCTATAGTGAGTCGTATTAGCTT | 712 |
| Adgrg6_15 | CGGCAAACTACGCGAGCCATAATCGTATATCGCCTTACGATAATAGAACCGAATATTCTTGCCGGGCTC AGAGTGTTCTCAAGCGTATATAGTGGTTACGCCGTTACATCAGTCCTATAGTGAGTCGTATTAGCTT | 713 |
| Adgrg6_16 | CGGCAAACTACGCGAGCCATAATCGTATATCGCCTTACGATAATAGAACCGAAAGGCCACTGCCAAGCC TAACTATGAGGTAGCGTATATAGTGGTTACGCCGTTACATCAGTCCTATAGTGAGTCGTATTAGCTT | 714 |
| Adgrg6_17 | CGGCAAACTACGCGAGCCATAATCGTATATCGCCTTACGATAATAGAACCGAAAATGGAGATGCTTAGG CTGCGTGTCTTGAGCGTATATAGTGGTTACGCCGTTACATCAGTCCTATAGTGAGTCGTATTAGCTT | 715 |
| Adgrg6_18 | CGGCAAACTACGCGAGCCATAATCGTATATCGCCTTACGATAATAGAACCGAAGCCACAGTCTGATCCA TGCATATTCCACAGCGTATATAGTGGTTACGCCGTTACATCAGTCCTATAGTGAGTCGTATTAGCTT | 716 |
| Adgrg6_19 | CGGCAAACTACGCGAGCCATAATCGTATATCGCCTTACGATAATAGAACCGAATTGGGCTCAACCTACA CCCTCACTAACAAGCGTATATAGTGGTTACGCCGTTACATCAGTCCTATAGTGAGTCGTATTAGCTT | 717 |
| Adgrg6_20 | CGGCAAACTACGCGAGCCATAATCGTATATCGCCTTACGATAATAGAACCGAAAGCATGCACTGAGGCC CATGAATAGGACAGCGTATATAGTGGTTACGCCGTTACATCAGTCCTATAGTGAGTCGTATTAGCTT | 718 |

**[Table 3-3]**

| Probe name | Base sequence (5' → 3') | SEQ ID NO: |
|---|---|---|
| Adgrg6_21 | CGGCAAACTACGCGAGCCATAATCGTATATCGCCTTACGATAATAGAACCGAAAAAGTTCAGGTCTGCC AAGATGGCAGTCAGCGTATATAGTGGTTACGCCGTTACATCAGTCCTATAGTGAGTCGTATTAGCTT | 719 |
| Adgrg6_22 | CGGCAAACTACGCGAGCCATAATCGTATATCGCCTTACGATAATAGAACCGAAAGGACACAGCAACTCT GATGTAGCTGGCAGCGTATATAGTGGTTACGCCGTTACATCAGTCCTATAGTGAGTCGTATTAGCTT | 720 |
| Adgrg6_23 | CGGCAAACTACGCGAGCCATAATCGTATATCGCCTTACGATAATAGAACCGAAGCTCTGCTGAGGTGAA TCTTAGTCACCTAGCGTATATAGTGGTTACGCCGTTACATCAGTCCTATAGTGAGTCGTATTAGCTT | 721 |
| Adgrg6_24 | CGGCAAACTACGCGAGCCATAATCGTATATCGCCTTACGATAATAGAACCGAACAGCTGTGTGTAGCCT GAACTCTGGGTCAGCGTATATAGTGGTTACGCCGTTACATCAGTCCTATAGTGAGTCGTATTAGCTT | 722 |
| Adgrg6_25 | CGGCAAACTACGCGAGCCATAATCGTATATCGCCTTACGATAATAGAACCGAACACATCACAGTGAAAG GAGAGGGTGGGAAGCGTATATAGTGGTTACGCCGTTACATCAGTCCTATAGTGAGTCGTATTAGCTT | 723 |
| Adgrg6_26 | CGGCAAACTACGCGAGCCATAATCGTATATCGCCTTACGATAATAGAACCGAATAGGAGAAAGCTGTCC AGTCACCACCTTAGCGTATATAGTGGTTACGCCGTTACATCAGTCCTATAGTGAGTCGTATTAGCTT | 724 |
| Adgrg6_27 | CGGCAAACTACGCGAGCCATAATCGTATATCGCCTTACGATAATAGAACCGAAACAAGCATCTTGAGCC AGAGATGGACAGAGCGTATATAGTGGTTACGCCGTTACATCAGTCCTATAGTGAGTCGTATTAGCTT | 725 |
| Adgrg6_28 | CGGCAAACTACGCGAGCCATAATCGTATATCGCCTTACGATAATAGAACCGAATGTCCTCTTTGTCCTT CACAGGTAACGCAGCGTATATAGTGGTTACGCCGTTACATCAGTCCTATAGTGAGTCGTATTAGCTT | 726 |
| Adgrg6_29 | CGGCAAACTACGCGAGCCATAATCGTATATCGCCTTACGATAATAGAACCGAACCACACAAGACAGAGC TGCTCCAAGTTTAGCGTATATAGTGGTTACGCCGTTACATCAGTCCTATAGTGAGTCGTATTAGCTT | 727 |

**[Table 4-1]**

| Probe name | Base sequence (5' → 3') | SEQ ID NO: |
|---|---|---|
| Vgf_1 | CGGCAAACTACGCGAGCCATACGCCTATTCGCCAATAAGACGAACTAGCGAAATGCCTCTACCTGAGCTA ATCCTTGCTGAATCGCTAGTGGTAAGTACGACTATTGAGACCACCTATAGTGAGTCGTATTAGCTT | 728 |
| Vgf_2 | CGGCAAACTACGCGAGCCATACGCCTATTCGCCAATAAGACGAACTAGCGAAACGCCCTGCAACAGATAC TGGAGCAGCAGATCGCTAGTGGTAAGTACGACTATTGAGACCACCTATAGTGAGTCGTATTAGCTT | 729 |
| Vgf_3 | CGGCAAACTACGCGAGCCATACGCCTATTCGCCAATAAGACGAACTAGCGAAATCGTTCTCCCGCTCTTG CTGCGTCTCCTATCGCTAGTGGTAAGTACGACTATTGAGACCACCTATAGTGAGTCGTATTAGCTT | 730 |
| Vgf_4 | CGGCAAACTACGCGAGCCATACGCCTATTCGCCAATAAGACGAACTAGCGAAATCATGACCAACGGGCTG CCAGAGACAGCATCGCTAGTGGTAAGTACGACTATTGAGACCACCTATAGTGAGTCGTATTAGCTT | 731 |
| Vgf_5 | CGGCAAACTACGCGAGCCATACGCCTATTCGCCAATAAGACGAACTAGCGAAATTCGTTCCAGTCCGGCA ACTCATCCCGGATCGCTAGTGGTAAGTACGACTATTGAGACCACCTATAGTGAGTCGTATTAGCTT | 732 |
| Vgf_6 | CGGCAAACTACGCGAGCCATACGCCTATTCGCCAATAAGACGAACTAGCGAAAAAACACCTCATCCTCCT CCCGATCCCAGATCGCTAGTGGTAAGTACGACTATTGAGACCACCTATAGTGAGTCGTATTAGCTT | 733 |
| Vgf_7 | CGGCAAACTACGCGAGCCATACGCCTATTCGCCAATAAGACGAACTAGCGAAAGTGTCCGCGGCCGAATG TAGTTTGGGAAATCGCTAGTGGTAAGTACGACTATTGAGACCACCTATAGTGAGTCGTATTAGCTT | 734 |
| Vgf_8 | CGGCAAACTACGCGAGCCATACGCCTATTCGCCAATAAGACGAACTAGCGAAACGCAGGTGGCAACGCGT GATGGAAGTGAATCGCTAGTGGTAAGTACGACTATTGAGACCACCTATAGTGAGTCGTATTAGCTT | 735 |
| Vgf_9 | CGGCAAACTACGCGAGCCATACGCCTATTCGCCAATAAGACGAACTAGCGAAAAGAAGAGGACGGATGCC GGCAACGTGAAATCGCTAGTGGTAAGTACGACTATTGAGACCACCTATAGTGAGTCGTATTAGCTT | 736 |
| Vgf_10 | CGGCAAACTACGCGAGCCATACGCCTATTCGCCAATAAGACGAACTAGCGAAAAGTCTGACAGGTCCGGT GCCGACAGAACATCGCTAGTGGTAAGTACGACTATTGAGACCACCTATAGTGAGTCGTATTAGCTT | 737 |

**[Table 4-2]**

| Probe name | Base sequence (5' → 3') | SEQ ID NO: |
|---|---|---|
| Vgf_11 | CGGCAAACTACGCGAGCCATACGCCTATTCGCCAATAAGACGAACTAGCGAAAGTCCTAGAAGCTGGGC TCAGCTGGGTCAATCGCTAGTGGTAAGTACGACTATTGAGACCACCTATAGTGAGTCGTATTAGCTT | 738 |
| Vgf_12 | CGGCAAACTACGCGAGCCATACGCCTATTCGCCAATAAGACGAACTAGCGAAATTTCCCGTCTGGGACT GGGAAGTCGCTAATCGCTAGTGGTAAGTACGACTATTGAGACCACCTATAGTGAGTCGTATTAGCTT | 739 |
| Vgf_13 | CGGCAAACTACGCGAGCCATACGCCTATTCGCCAATAAGACGAACTAGCGAAAGAGAAGGAGAAGTGGG TAAGTTCACAGCATCGCTAGTGGTAAGTACGACTATTGAGACCACCTATAGTGAGTCGTATTAGCTT | 740 |
| Vgf_14 | CGGCAAACTACGCGAGCCATACGCCTATTCGCCAATAAGACGAACTAGCGAAATTTGGCCGGGACACTG CGTCCTCAGCTAATCGCTAGTGGTAAGTACGACTATTGAGACCACCTATAGTGAGTCGTATTAGCTT | 741 |
| Vgf_15 | CGGCAAACTACGCGAGCCATACGCCTATTCGCCAATAAGACGAACTAGCGAAAATTCCGAGCAGCTCGG ACCTCTGGTACGATCGCTAGTGGTAAGTACGACTATTGAGACCACCTATAGTGAGTCGTATTAGCTT | 742 |
| Vgf_16 | CGGCAAACTACGCGAGCCATACGCCTATTCGCCAATAAGACGAACTAGCGAAATGGAAGAGCTCTCCCT GGTCCTGAGGCTATCGCTAGTGGTAAGTACGACTATTGAGACCACCTATAGTGAGTCGTATTAGCTT | 743 |
| Vgf_17 | CGGCAAACTACGCGAGCCATACGCCTATTCGCCAATAAGACGAACTAGCGAAAGACGGTCCAGTGCCTG CAACAGTACCGAATCGCTAGTGGTAAGTACGACTATTGAGACCACCTATAGTGAGTCGTATTAGCTT | 744 |
| Vgt_18 | CGGCAAACTACGCGAGCCATACGCCTATTCGCCAATAAGACGAACTAGCGAAAGAGCTTCTGCTGCTTC TTCGGGTGTTCCATCGCTAGTGGTAAGTACGACTATTGAGACCACCTATAGTGAGTCGTATTAGCTT | 745 |
| Vgf_19 | CGGCAAACTACGCGAGCCATACGCCTATTCGCCAATAAGACGAACTAGCGAAACTGGAGTATGAGCGAT GGTCGAGGGCTGATCGCTAGTGGTAAGTACGACTATTGAGACCACCTATAGTGAGTCGTATTAGCTT | 746 |
| Vgf_20 | CGGCAAACTACGCGAGCCATACGCCTATTCGCCAATAAGACGAACTAGCGAAAGCTTGGATTTCTGGTG CCGGGAGGCTATATCGCTAGTGGTAAGTACGACTATTGAGACCACCTATAGTGAGTCGTATTAGCTT | 747 |
| Vgf_21 | CGGCAAACTACGCGAGCCATACGCCTATTCGCCAATAAGACGAACTAGCGAAAAAGGATGCTAGCGCCT CCAGCTCTTCTGATCGCTAGTGGTAAGTACGACTATTGAGACCACCTATAGTGAGTCGTATTAGCTT | 748 |
| Vgt_22 | CGGCAAACTACGCGAGCCATACGCCTATTCGCCAATAAGACGAACTAGCGAAACTCTTGTTGGCGCTTA GCATTGCTCGGAATCGCTAGTGGTAAGTACGACTATTGAGACCACCTATAGTGAGTCGTATTAGCTT | 749 |
| Vgf_23 | CGGCAAACTACGCGAGCCATACGCCTATTCGCCAATAAGACGAACTAGCGAAATCAGCGTGTGCGTGCG GGTTTCAGTCTCATCGCTAGTGGTAAGTACGACTATTGAGACCACCTATAGTGAGTCGTATTAGCTT | 750 |
| Vgf_24 | CGGCAAACTACGCGAGCCATACGCCTATTCGCCAATAAGACGAACTAGCGAAAGCTTTCGGACATTCGA GCCTGGAATTGGATCGCTAGTGGTAAGTACGACTATTGAGACCACCTATAGTGAGTCGTATTAGCTT | 751 |

Next, after unhybridized capturing probes were washed away by washing, the three types of probes shown in the above-described Table 1 were added as detection probes, and the sample was incubated at 42°C for 16 hours for hybridization. Subsequently, unhybridized detection probes were washed away by washing, and then the sample was observed by using a confocal microscope (Leica DMi8). The results are shown in FIG. 1 (image captured by using an objective lens (HC PLAPO 100× NA 1.47)).

As shown in FIG. 1, it was verified that mRNAin mouse ES cells can be visualized by using the above-described three kinds of detection probes.

### [Industrial Applicability]

The probe of the present embodiment has a base sequence that is not present in the genome of a mammal and can be utilized to simultaneously detect a large number of various nucleic acids of a mammal.

## Claims

1. A probe comprising:
a base sequence that does not hybridize with a nucleic acid of a mammal under stringent conditions,
wherein the probe has a sequence including a base sequence set forth in any of SEQ ID NOs:1 to 673.

2. The probe according to Claim 1,
wherein the mammal is one or more kinds of animals selected from the group consisting of a human and a mouse.

3. The probe according to Claim 1 or 2,
wherein the mammal is one or more kinds of animals selected from the group consisting of a human, a mouse, a zebrafish, an African clawed frog, and a rat, and
the probe has a sequence including a base sequence set forth in any of SEQ ID NOs:6, 22, 31, 42, 43, 54, 61, 71, 86 to 88, 91, 109, 114, 118, 125, 128, 134, 140, 141, 146, 149, 151, 157, 167, 201, 213, 214, 218, 220, 221, 228, 230, 234, 241, 242, 248, 253, 283, 289, 294, 298, 303, 305, 310, 327, 349, 363, 365, 375, 378, 385, 394, 400, 406, 412, 416, 418, 420, 421, 430, 431, 439, 475, 501, 523, 532, 548, 557, 563, 585, 589, 593, 608, 620, 631, 642, and 646.

4. The probe according to Claim 1 or 2, further comprising:
a marker substance.

5. A probe set comprising:
two or more kinds of the probes according to Claim 1 or 2,
wherein the two or more kinds of the probes do not hybridize with each other under stringent conditions.

6. A nucleic acid detection kit for mammals, the nucleic acid detection kit comprising:
one or more kinds of detection probes including the probe according to Claim 4; and
one or more kinds of capturing probes each having a sequence A complementary to at least a portion of the detection probes and a sequence B complementary to at least a portion of a target nucleic acid of a mammal,
wherein in a case where the nucleic acid detection kit includes two or more kinds of the detection probes, the two or more kinds of the detection probes do not hybridize with each other under stringent conditions, and
in a case where the nucleic acid detection kit includes two or more kinds of the capturing probes, the two or more kinds of the capturing probes do not hybridize with each other under stringent conditions.

7. A method for simultaneously detecting a plurality of nucleic acids of a mammal, using the nucleic acid detection kit for mammals according to Claim 6, the method comprising:
bringing two or more kinds of the capturing probes into contact with a cell or a tissue of a mammal;
removing the unhybridized capturing probes by washing and then bringing two or more kinds of the detection probes into contact with a cell or a tissue of a mammal; and
removing the unhybridized detection probes by washing and then detecting the detection probes.
